# EUROPEAN PATENT APPLICATION

(11) **EP 3 804 757 A1**
(43) Date of publication of application: **14.04.2021**
(21) Application number: 19810489.5
(22) Date of filing: 24.05.2019
(51) Int. Cl.: A61K 45/00, A61K 31/436, A61K 31/519, A61K 45/06, A61P 1/04, A61P 35/00, A61P 43/00

(54) **TREATMENT AGENT FOR DIFFUSE GASTRIC CANCER**

(30) Priority: 26.05.2018 JP 2018101086
(71) Applicant: Kanazawa Medical University, Uchinada-machi Kahoku-gun Ishikawa 920-0293 (JP)
(72) Inventor: YASUMOTO Kazuo, Kahoku-gun, Ishikawa 920-0293 (JP)
(74) Representative: Hoffmann Eitle
(86) International application number: PCT/JP2019/020689
(87) International publication number: WO 2019/230595

(57) **Abstract**

To provide a therapeutic agent to achieve highly effect on diffuse gastric cancer typified by scirrhous gastric cancer and a therapeutic agent to achieve highly effect on cMet gene-amplified gastric cancer. The problem is solved by a therapeutic agent for treating diffuse gastric cancer containing (i) an MEK inhibitor or (ii) an MEK inhibitor and an mTOR inhibitor or a therapeutic agent for treating cMet gene-amplified gastric cancer containing (i) an MEK inhibitor or (ii) an MEK inhibitor and an mTOR inhibitor.

## Description

### Technical Field

The present invention relates to a method of treating mammalian diffuse gastric cancer and a pharmaceutical composition useful for such treatment. In particular, the method relates to a method of using a pharmaceutical composition containing an MEK inhibitor or a pharmaceutical composition containing an MEK inhibitor and an mTOR inhibitor for treating diffuse gastric cancer or cMet gene-amplified gastric cancer.

### Background Art

Diffuse gastric cancer, typified by scirrhous gastric cancer, includes poorly differentiated adenocarcinoma and signet-ring cell carcinoma, grows fast, is likely to develop into peritoneal dissemination (peritoneal carcinomatosis) with malignant ascites, which is difficult to treat, and is thought to have poor prognosis as compared with non-diffuse gastric cancer including well-differentiated adenocarcinoma. In particular, the scirrhous gastric cancer is a refractory gastric cancer characterized by diffuse infiltrative growth of cancer cells, hyperplasia of cancer stromal fibroblasts, and onset of peritoneal carcinomatosis frequently complicated with malignant ascites (Non-Patent Document 1). These characteristics coincide with the fact that 50 to 60% of the progressive gastric cancer is diffuse gastric cancer, about 20% of which is considered to be scirrhous gastric cancer, and 50 to 60% of the recurrent gastric cancer is peritoneal carcinomatosis. It is also known that the condition of peritoneal carcinomatosis once developing into malignant ascites progresses and becomes worse in a short term (Non-Patent Document 2, Non-Patent Document 3).

Due to the feature of diffuse gastric cancer growing fast, most of the progressive cases are diagnosed in stage IV with malignant ascites and cannot be expected to be treated by radical surgical resection (Non-Patent Document 2, Non-Patent Document 3). Hence, treatment with anticancer drugs such as CDDP, S-1, Capecitabin, oxaliplatin (first-line), paclitaxel, ramucirumab (anti-VEGFR2 antibody), CPT-11, and Nivolumab (anti-PD-1 antibody) (second-line) is performed but provides poor therapeutic effect, compared with the cases of non-diffuse gastric cancer. The expression of human epidermal growth factor receptor 2 (HER2), which is the only molecular target for the gastric cancer, is not observed in the diffuse gastric cancer. Hence, targeted therapy using the anti-HER2 antibody or the like cannot be applied. Well-differentiated adenocarcinoma included in non-diffuse gastric cancer frequently has genetic abnormalities and thus is likely to be targeted by immunotherapy, whereas diffuse gastric cancer has few genetic abnormalities and low immunogenicity and thus is scarcely considered to be a target of immunotherapy (Non-Patent Document 6).

Analysis of cancer development mechanisms which are typical of the diffuse gastric cancer has revealed that cancer stromal fibroblasts that are abundant specific for this pathology highly produce hepatocyte growth factor (hereinafter called HGF) (Non-Patent Document 1). Such cancer stroma-derived HGF enhances the mobility of diffuse gastric cancer cells and also induces potent cell proliferation activity, through cMet, the only receptor for HGF (Non-Patent Document 1). In addition, such paracrine-inducible HGF induces, from diffuse gastric cancer cells, production of a large amount of amphiregulin (hereinafter also called AP) (Non-Patent Document 3) that is an EGFR ligand having potent cell proliferation activity as with HGF (Non-Patent Document 1). The malignant ascites complicated by diffuse gastric cancer contains 2 to 5 times or more higher concentrations of HGF and AP than in non-malignant ascites (Non-Patent Document 3). Stroma-induced HGF and self-derived AP strongly cause proliferation of the diffuse gastric cancer cells in a paracrine manner and an autocrine manner, respectively. In contrast, in non-diffuse gastric cancer, potent induction of HGF production from cancer stromal fibroblasts is not observed unlike cancer stroma of diffuse gastric cancer. In addition, HGF or AP stimulation does not have any cell proliferation-inducing activity on non-diffuse gastric cancer cells. Such evidences greatly differentiate the non-diffuse gastric cancer cells from the diffuse gastric cancer cell (Non-Patent Document 1). In the diffuse gastric cancer, it is considered that activation of cancer stroma-induced paracrine HGF through cMet receptor (HGF/cMet axis pathway) may play an important role (Non-Patent Document 1), and it is also reported that relations between cMet gene amplification and onset of scirrhous gastric cancer and diffuse gastric cancer were exhibited in the gastric cancer cells (Non-Patent Document 7). A cMet inhibitor has exhibited potent antitumor effect in mouse peritoneal carcinomatosis models using cMet gene-amplified gastric cancer or scirrhous gastric cancer cells (Non-Patent Document 1). Meanwhile, some clinical trials have been carried out on general gastric cancers by using a TKI (tyrosine kinase inhibitor) or an antibody which target to the cMet receptor, but insufficient clinical efficacy has been identified in each trial, unfortunately.

Mitogen-activated protein (MAP) kinase/extracellular signal-regulated kinase (ERK) kinase and MEK are known to be involved in regulation of cell proliferation as kinases that mediate Raf-MEK-ERK signal transduction cascade. Raf family (such as B-Raf and C-Raf) activates MEK family (such as MEK-1 and MEK-2), and the MEK family activates ERK family (such as ERK-1 and ERK-2) (Non-Patent Document 4, FIG. 1).

MEK inhibitory activity is known to effectively induce inhibition of ERK1/2 activity and suppression of cell proliferation (Non-Patent Document 5).

In addition to the above Raf-MEK-ERK signal transduction cascade, PI3K/AKT/mTOR signal transduction cascade is also known as a pathway involved in cancer development. Mammalian target of rapamycin (mTOR) is serine/threonine kinase that regulates cell growth, cell proliferation, cell mobility, cell survival, protein synthesis, and transcription.

Stimulation of a growth factor causes activation of PI3K/AKT/mTOR signal transduction cascade, and mTOR affects cell division, cell death, angiogenesis, energy production, and the like to promote cancer cell growth. Hence, the PI3K/AKT/mTOR signal transduction cascade is considered important as a mechanism of promoting the proliferation of cancer cells or sarcoma cells (Non-Patent Document 4, FIG. 1).

### Prior Art Documents

### Non-Patent Documents

Non-Patent Document 1: Cancer Science, December 2013, vol. 104, No. 12, pp.1640-1646
Non-Patent Document 2: Cancer Res. 2006; 66: (4) pp. 2181-2187, February 15, 2006
Non-Patent Document 3: Clin. Cancer Res. 2011; 17: 3619-3630
Non-Patent Document 4: Modern Media, vol. 61, No. 8, pp. 18-22, 2015
Non-Patent Document 5: The Journal of Biological Chemistry, vol. 276, No. 4, pp. 2686-2692, 2001
Non-Patent Document 6 : Cancer genome landscapes, Science 39 (6127):1546-8
Non-Patent Document 7: Cancer 1999; 85: 1894-1902

### Summary of the Invention

### Problems that the Invention is to Solve

As described above, diffuse gastric cancer and non-diffuse gastric cancer have different molecular mechanisms in development and growth, and thus a therapeutic agent achieving high antitumor effect on non-diffuse gastric cancer is not expected to produce sufficient effect as an therapeutic agent for treating diffuse gastric cancer. There is therefore a need to develop a therapeutic agent that regulates diffuse gastric cancer on the basis of the understanding of the mechanisms of cell proliferation and cancer development specific to diffuse gastric cancer and achieves high antitumor effect on diffuse gastric cancer.

Whether the gastric cancer is diffuse gastric cancer or non-diffuse gastric cancer has been identified by macroscopic morphology. A cMet gene-amplified gastric cancer, which accounts for 2% of the gastric cancer cases, may be difficult to be classified as diffuse gastric cancer from the viewpoint of macroscopic morphology, but has been revealed to have the feature of diffuse gastric cancer from the viewpoint of the molecular mechanism of growth and development (Non-Patent Document 7).

### Means for solving the Problems

The inventors of the present invention have found that use of an MEK inhibitor exhibits remarkable therapeutic effect on both diffuse gastric cancer and cMet gene-amplified gastric cancer in the case of being administered as a single agent and combined use of an mTOR inhibitor with the MEK inhibitor exhibits more remarkable, synergistic therapeutic effect, and, therefore, have completed the present invention.

In other words, the present invention relates to the following embodiments.
[1] A therapeutic agent for treating diffuse gastric cancer, which comprises an MEK inhibitor;
[2] The therapeutic agent according to [1], wherein the MEK inhibitor is trametinib, a pharmacologically acceptable salt thereof, or a solvate thereof;
[3] The therapeutic agent according to [1] or [2], wherein the MEK inhibitor is a trametinib dimethyl sulfoxide;
[4] A therapeutic agent for treating diffuse gastric cancer, which comprises an MEK inhibitor and an mTOR inhibitor;
[5] The therapeutic agent according to [4], wherein the MEK inhibitor is trametinib, a pharmacologically acceptable salt thereof, or a solvate thereof;
[6] The therapeutic agent according to [4] or [5], wherein the MEK inhibitor is a trametinib dimethyl sulfoxide;
[7] The therapeutic agent according to any one of [4] to [6], wherein the mTOR inhibitor is everolimus, a pharmacologically acceptable salt thereof, or a solvate thereof;
[8] The therapeutic agent according to any one of [4] to [7], wherein the mTOR inhibitor is everolimus;
[9] A therapeutic agent for treating diffuse gastric cancer, which comprises an MEK inhibitor, in which the therapeutic agent is to be administered in combination with an mTOR inhibitor;
[10] The therapeutic agent according to [9], wherein the MEK inhibitor is trametinib, a pharmacologically acceptable salt thereof, or a solvate thereof;
[11] The therapeutic agent according to [9] or [10], wherein the MEK inhibitor is a trametinib dimethyl sulfoxide;
[12] The therapeutic agent according to any one of [9] to [11], wherein the mTOR inhibitor is everolimus, a pharmacologically acceptable salt thereof, or a solvate thereof;
[13] The therapeutic agent according to any one of [9] to [12], wherein the mTOR inhibitor is everolimus;
[14] The therapeutic agent according to any one of the aspects [1] to [13], wherein the diffuse gastric cancer is scirrhous gastric cancer;
[15] A therapeutic agent for treating cMet gene-amplified gastric cancer, which comprises an MEK inhibitor;
[16] The therapeutic agent according to [15], wherein the MEK inhibitor is trametinib, a pharmacologically acceptable salt thereof, or a solvate thereof; and
[17] The therapeutic agent according to [15] or [16], wherein the MEK inhibitor is a trametinib dimethyl sulfoxide.

### Advantageous Effect of the Invention

The present invention provides a therapeutic agent for treating diffuse gastric cancer which comprises an MEK inhibitor, a therapeutic agent for treating a cMet gene-amplified gastric cancer which comprises an MEK inhibitor, and a therapeutic agent for treating diffuse gastric cancer which comprises an MEK inhibitor and an mTOR inhibitor. Such a therapeutic agent can be used for treating diffuse gastric cancer.

### Brief Description of the Drawings

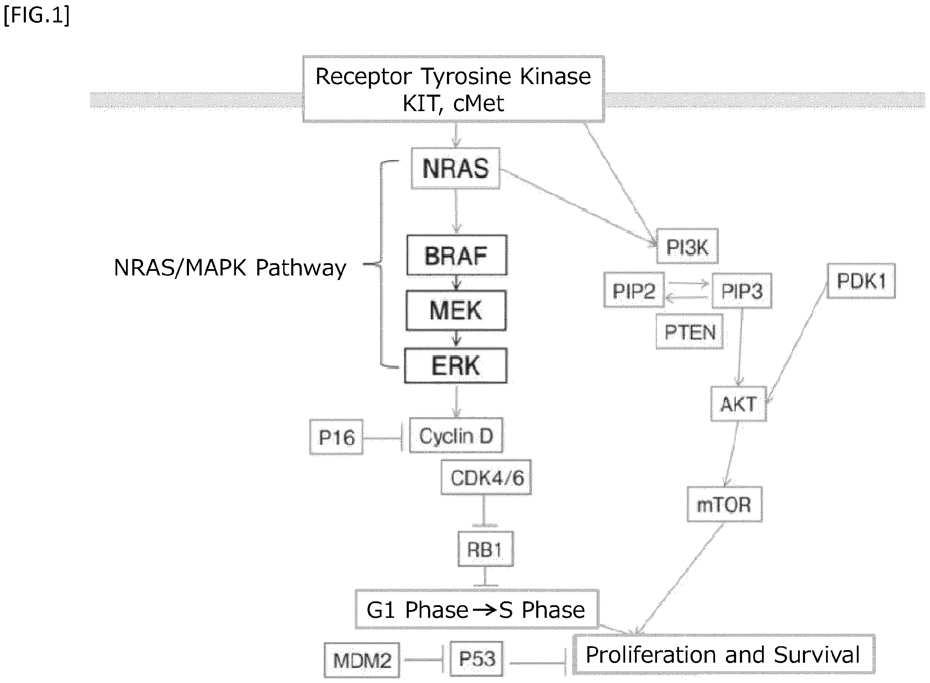

FIG. 1 shows intracellular signal transduction cascade.
FIG. 2 shows antitumor effects of trametinib on diffuse gastric cancer cells.

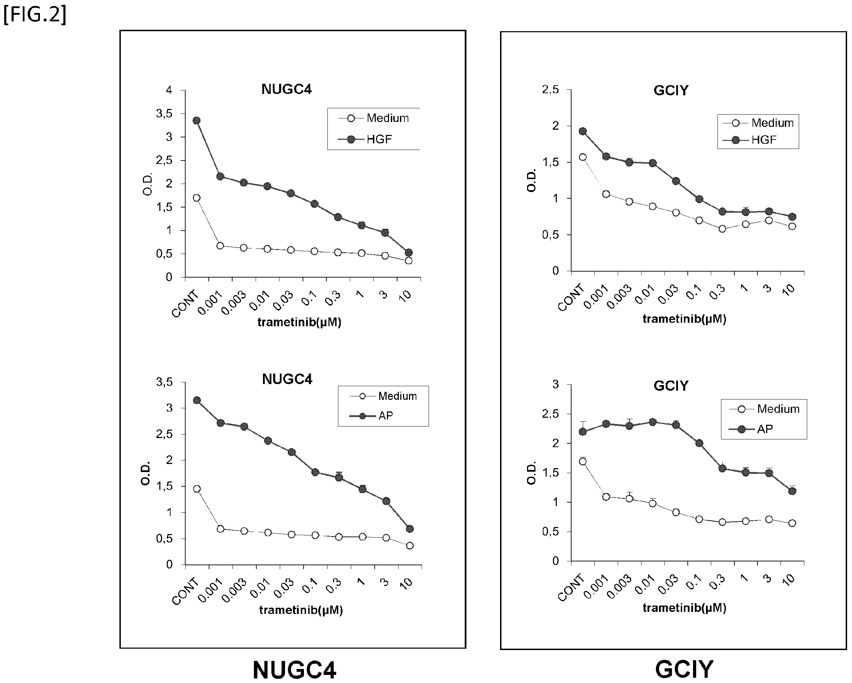

   In FIG. 2, "HGF" represents cell proliferation when HGF and trametinib were added; "AP" represents cell proliferation when amphiregulin and trametinib were added; and "Medium" represents cell proliferation when trametinib was added without HGF and AP (base line proliferation).

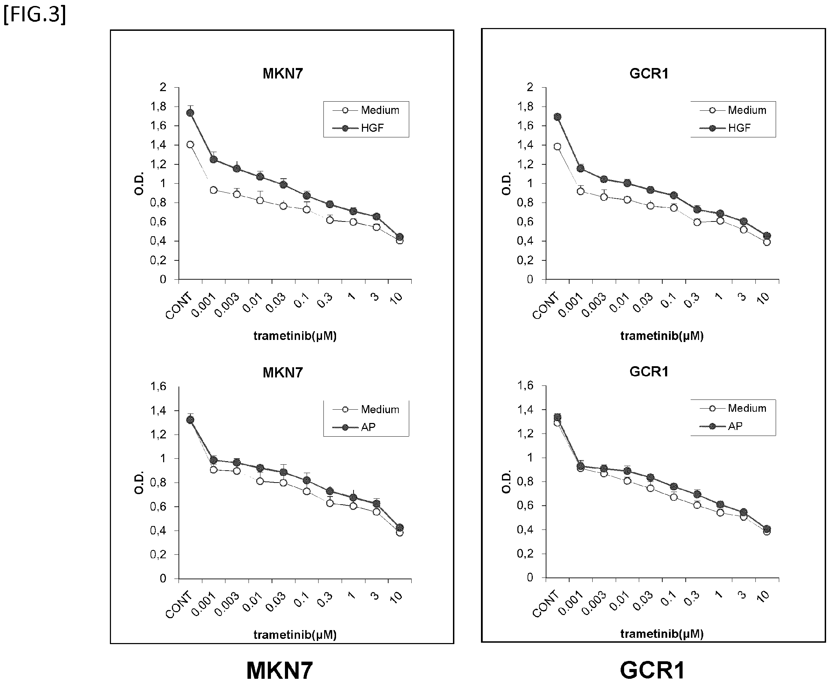

FIG. 3 shows antitumor effects of trametinib on non-diffuse gastric cancer cells. The meaning of each symbol is the same as each one in FIG. 2.

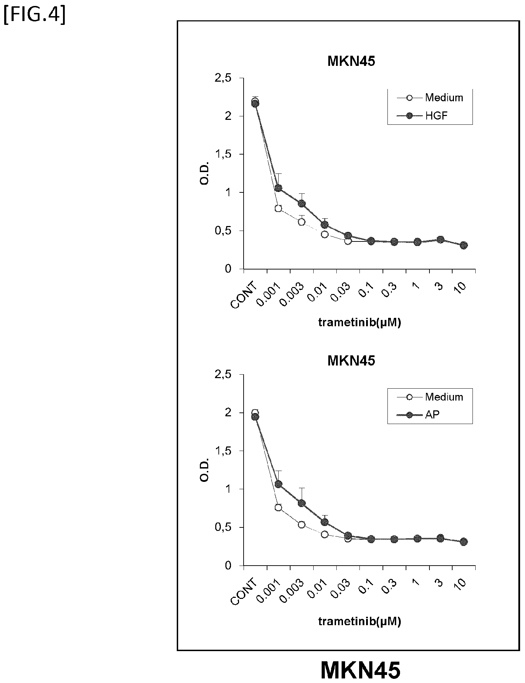

FIG. 4 shows antitumor effects of trametinib on cMet gene-amplified gastric cancer cells. The meaning of each symbol is the same as each one in FIG. 2.

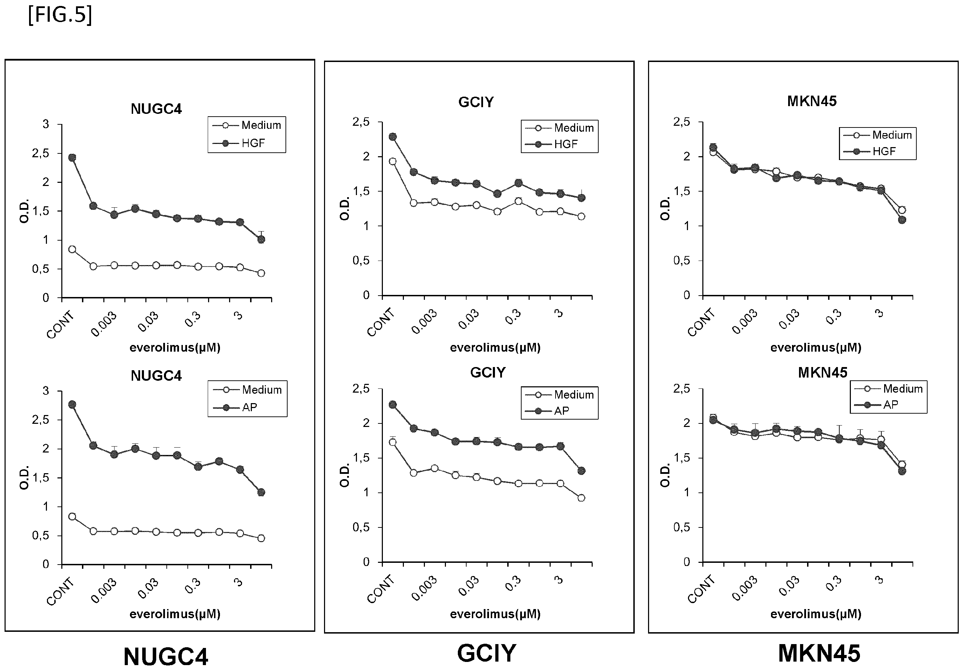

FIG. 5 shows antitumor effects of everolimus on diffuse gastric cancer cells or cMet gene-amplified gastric cancer cells.
   In FIG. 5, "HGF" represents cell proliferation when HGF and everolimus were added; "AP" represents cell proliferation when amphiregulin and everolimus were added; and "Medium" represents cell proliferation when everolimus was added without HGF and AP (base line proliferation).

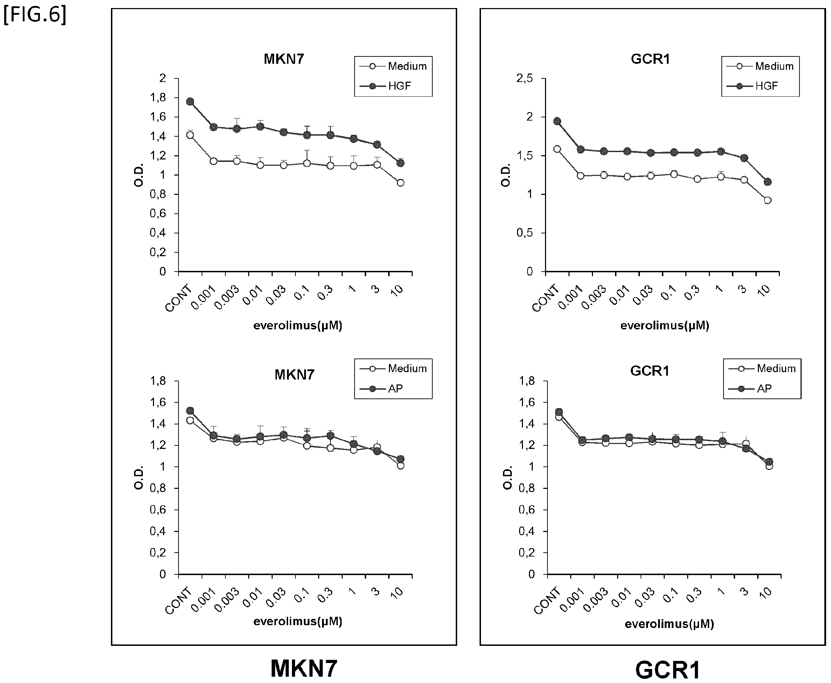

FIG. 6 shows antitumor effects of everolimus on non-diffuse gastric cancer cells. The meaning of each symbol is the same as each one in FIG. 5.

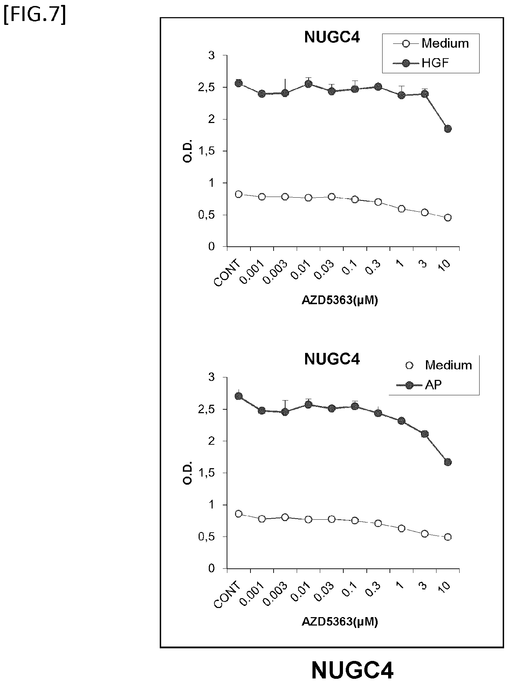

FIG. 7 shows antitumor effects of an Akt inhibitor, AZD5363, on diffuse gastric cancer cells.
   In FIG. 7, "HGF" represents cell proliferation when HGF and AZD5363 were added; "AP" represents cell proliferation when amphiregulin and AZD5363 were added; and "Medium" represents cell proliferation when AZD5363 was added without HGF and AP (base line proliferation).

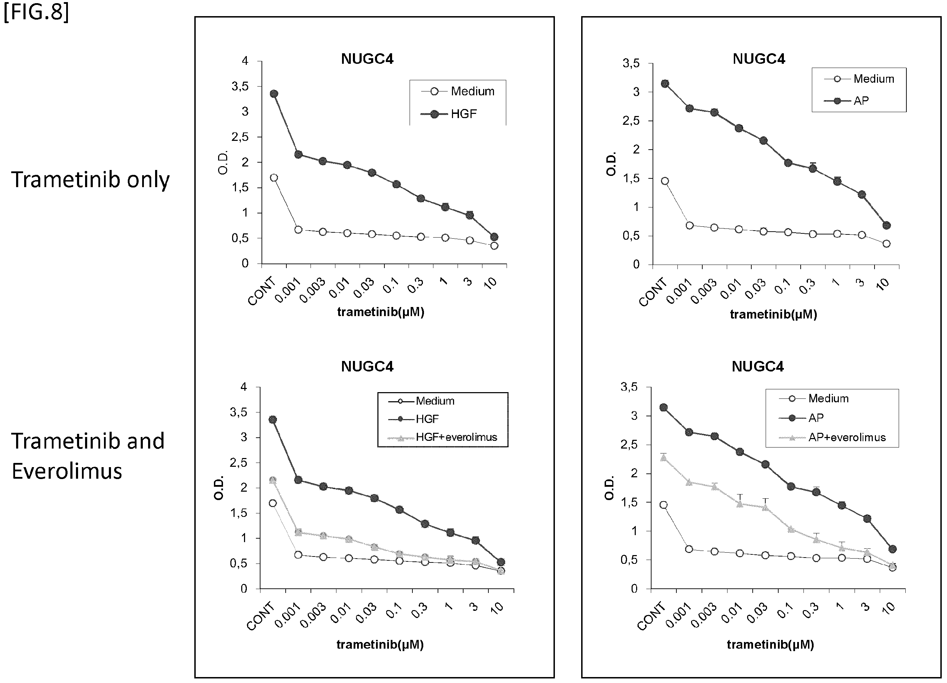

FIG. 8 shows synergistic cell proliferation inhibitory effects of trametinib and everolimus on diffuse gastric cancer cells.
   In the upper graphs (showing the effects of trametinib alone), "HGF" represents cell proliferation when HGF and trametinib were added; "AP" represents cell proliferation when amphiregulin and trametinib were added; and "Medium" represents cell proliferation when trametinib was added without HGF and AP (base line proliferation).
   In the lower graphs (showing combined effects of trametinib and everolimus), "HGF + everolimus" represents cell proliferation when HGF, trametinib and everolimus were added; "AP + everolimus" represents cell proliferation when amphiregulin, trametinib and everolimus were added; "HGF" represents cell proliferation when HGF and trametinib were added; "AP" represents cell proliferation when amphiregulin and trametinib were added; and "Medium" represents cell proliferation when trametinib was added without HGF and AP (base line proliferation).

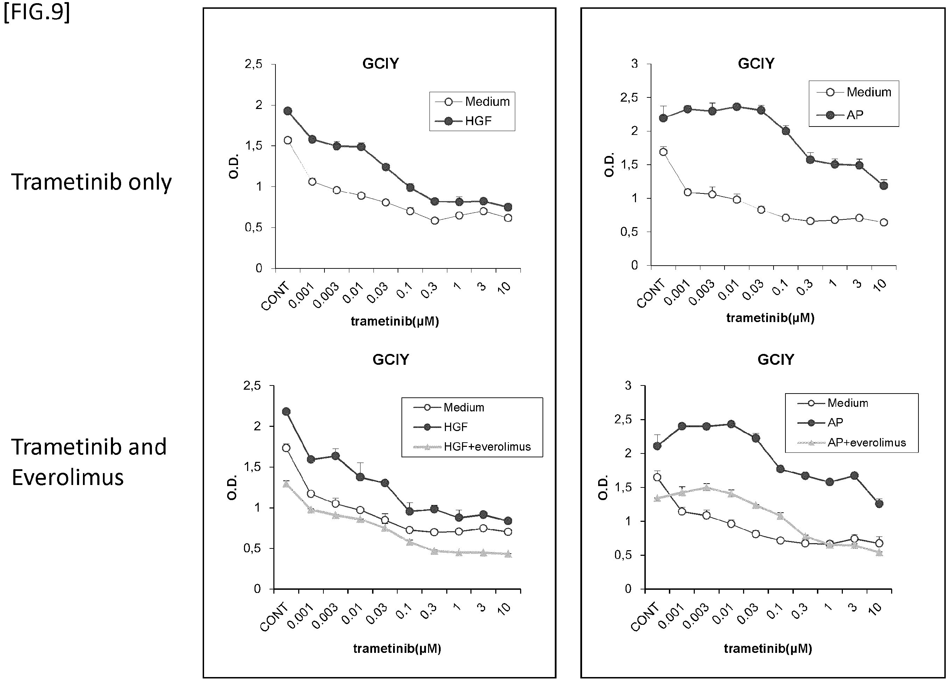

FIG. 9 shows synergistic cell proliferation inhibitory effects of trametinib and everolimus on diffuse gastric cancer cells. The meaning of each symbol is the same as each one in FIG. 8.

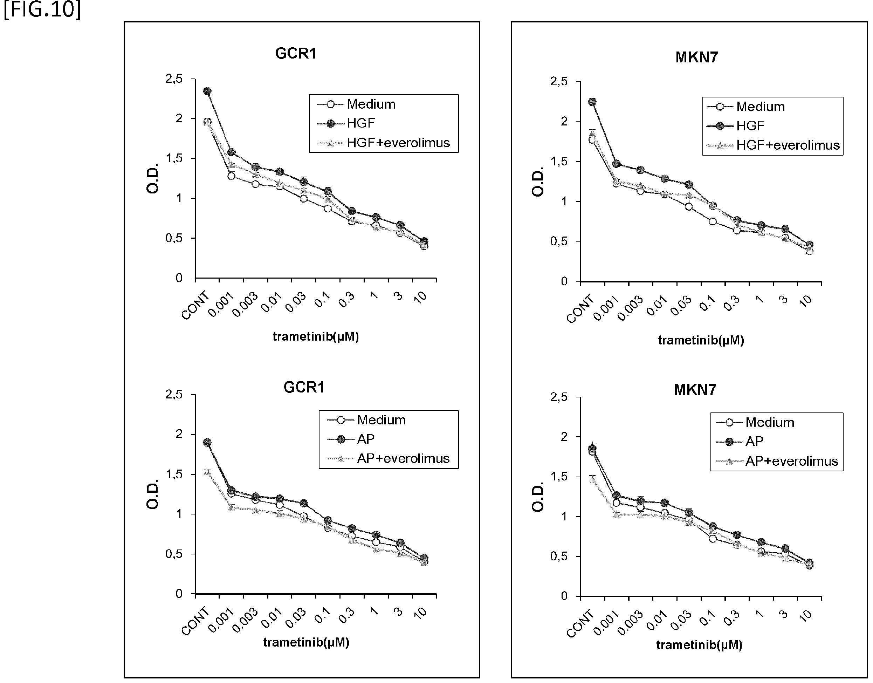

FIG. 10 shows cell proliferation inhibitory effects of trametinib and everolimus on non-diffuse gastric cancer cells. The meaning of each symbol is the same as each one in the lower graphs in FIG. 8.

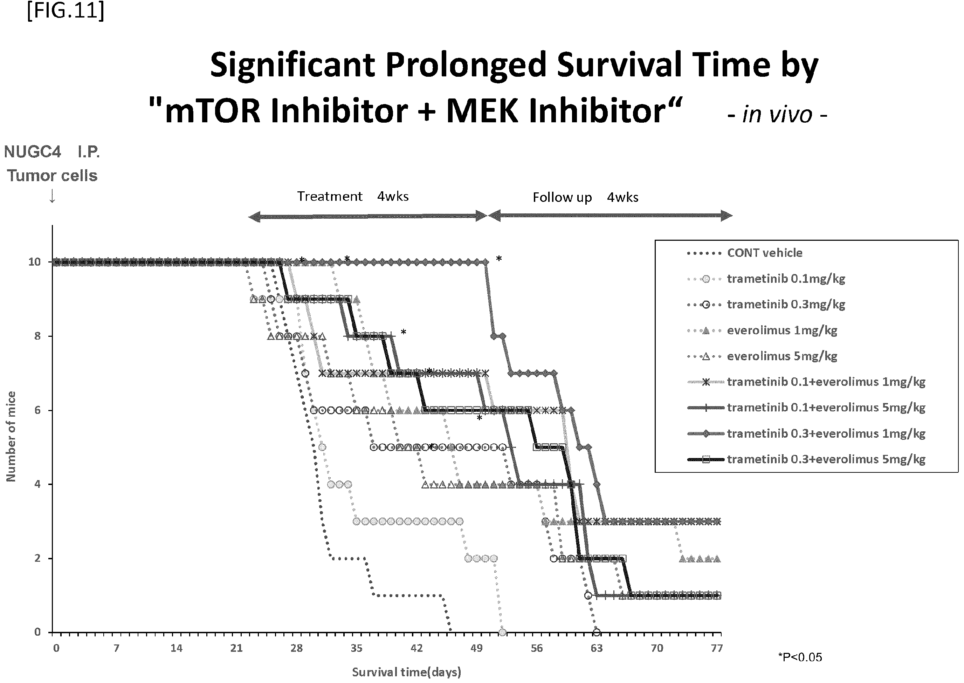

FIG. 11 shows prolonged survival times of Balb/c nu/nu mice transplanted with diffuse gastric cancer cells, based on synergistic cell proliferation inhibitory effects of trametinib and everolimus.

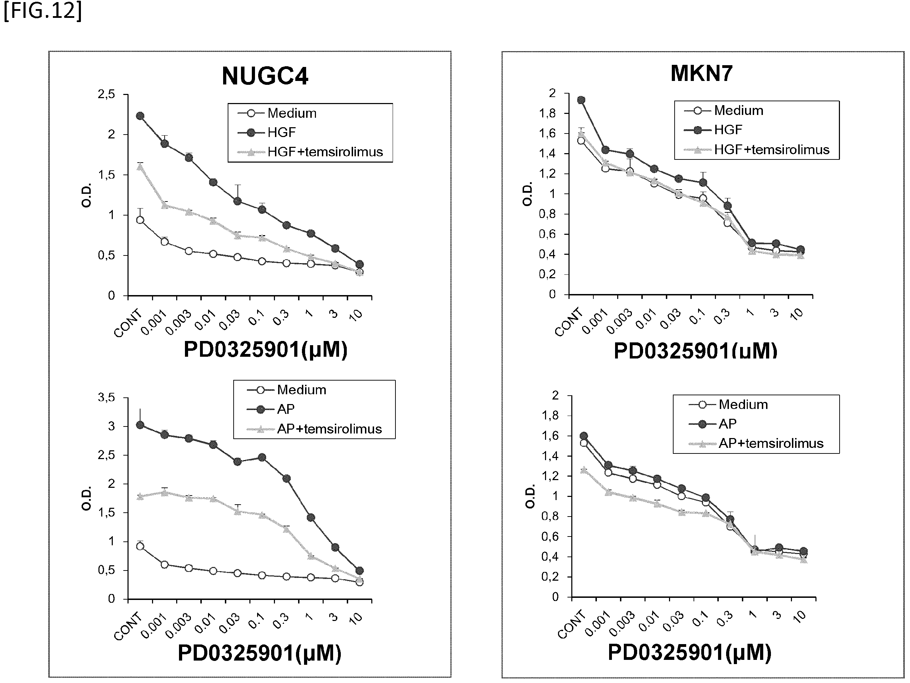

FIG. 12 shows proliferation inhibitory effects of combined use of PD0325901 (MEK inhibitor) and temsirolimus (mTOR inhibitor) on diffuse gastric cancer cells and non-diffuse gastric cancer cells. "HGF" represents cell proliferation when HGF and PD0325901 were added; "AP" represents cell proliferation when amphiregulin and PD0325901 were added; "Medium" represents cell proliferation when PD0325901 was added without HGF and AP (base line proliferation); "HGF + temsirolimus" represents cell proliferation when HGF, PD0325901 and temsirolimus were added; and "AP + temsirolimus" represents cell proliferation when amphiregulin, PD0325901 and temsirolimus were added.

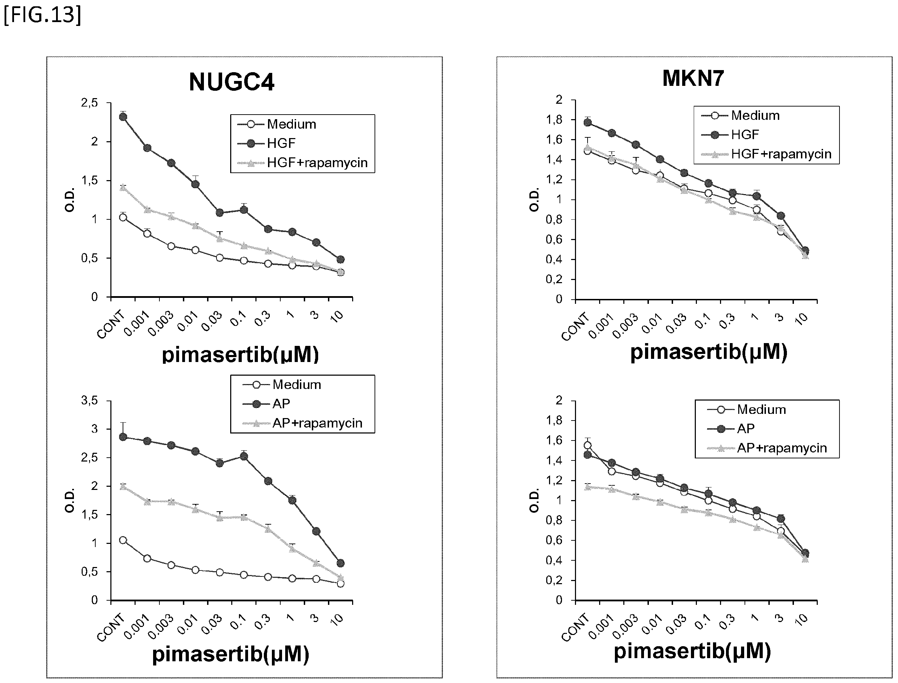

FIG. 13 shows proliferation inhibitory effects of combined use of pimasertib (MEK inhibitor) and rapamycin (mTOR inhibitor) on diffuse gastric cancer cells or non-diffuse gastric cancer cells. "HGF" represents cell proliferation when HGF and pimasertib were added; "AP" represents cell proliferation when amphiregulin and pimasertib were added; "Medium" represents cell proliferation when pimasertib was added without HGF and AP (base line proliferation); "HGF + rapamycin" represents cell proliferation when HGF, pimasertib and rapamycin were added; and "AP + rapamycin" represents cell proliferation when amphiregulin, pimasertib and rapamycin were added.

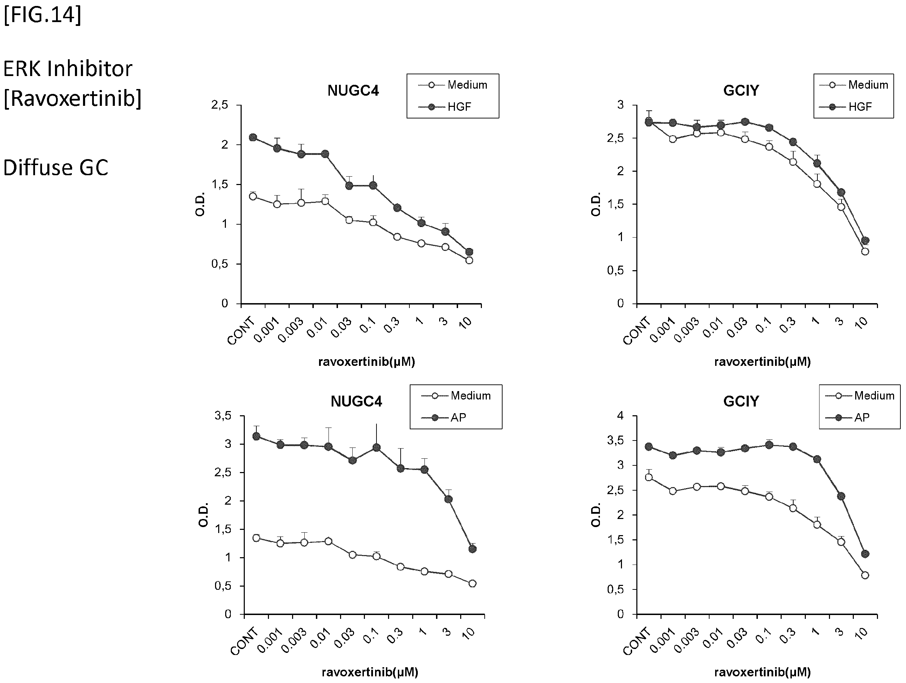

FIG. 14 shows proliferation inhibitory effects of ravoxertinib (ERK inhibitor) on diffuse gastric cancer cells. "HGF" represents cell proliferation when HGF and ravoxertinib were added; "AP" represents cell proliferation when amphiregulin and ravoxertinib were added; and "Medium" represents cell proliferation when ravoxertinib was added without HGF and AP (base line proliferation).

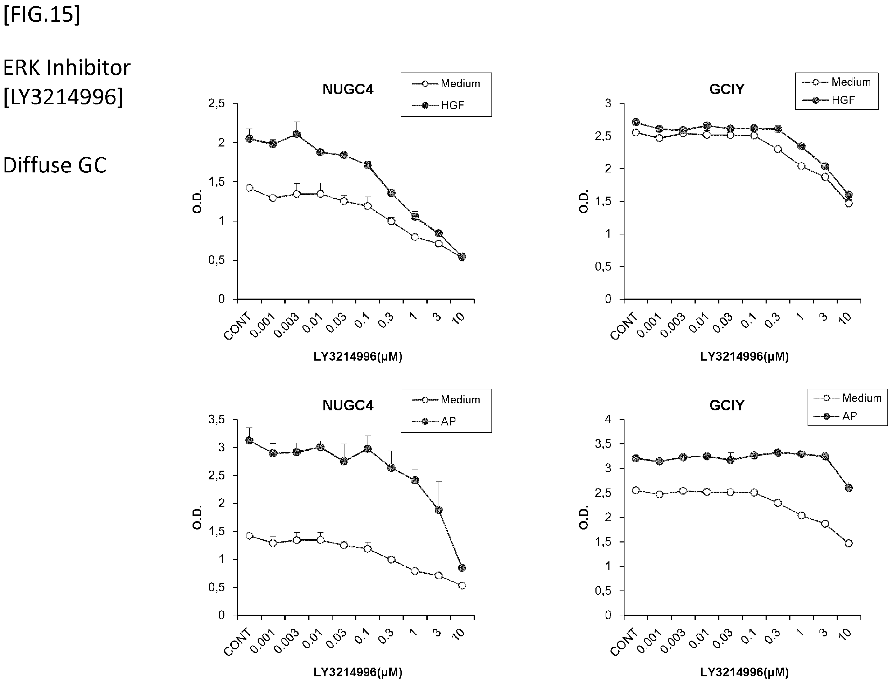

FIG. 15 shows proliferation inhibitory effects of LY3214996 (ERK inhibitor) on diffuse gastric cancer cells. "HGF" represents cell proliferation when HGF and LY3214996 were added; "AP" represents cell proliferation when amphiregulin and LY3214996 were added; and "Medium" represents cell proliferation when LY3214996 was added without HGF and AP (base line proliferation).

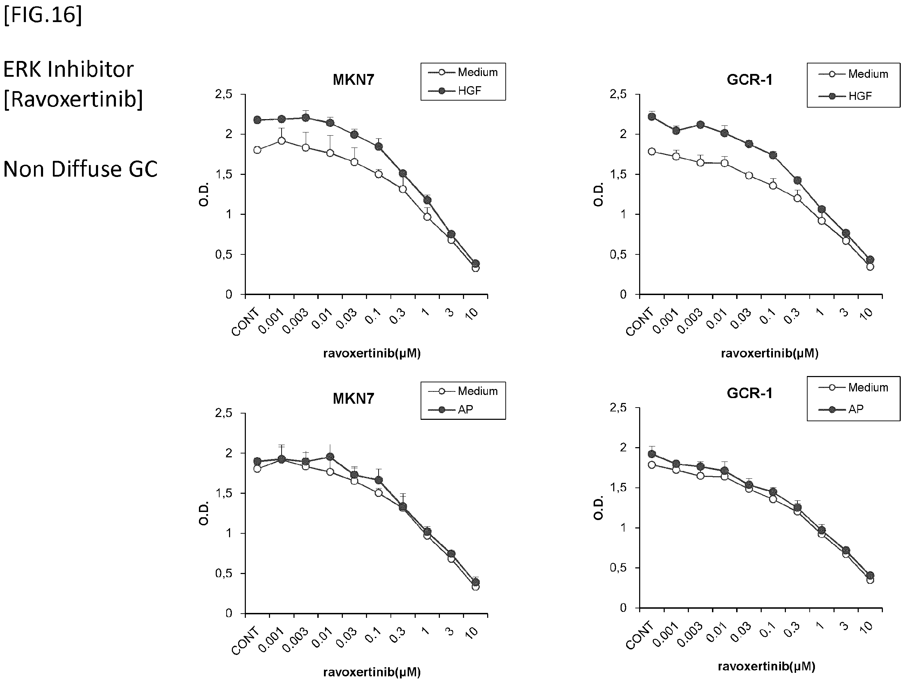

FIG. 16 shows proliferation inhibitory effects of ravoxertinib (ERK inhibitor) on non-diffuse gastric cancer cells. "HGF" represents cell proliferation when HGF and ravoxertinib were added; "AP" represents cell proliferation when amphiregulin and ravoxertinib were added; and "Medium" represents cell proliferation when ravoxertinib was added without HGF and AP (base line proliferation).

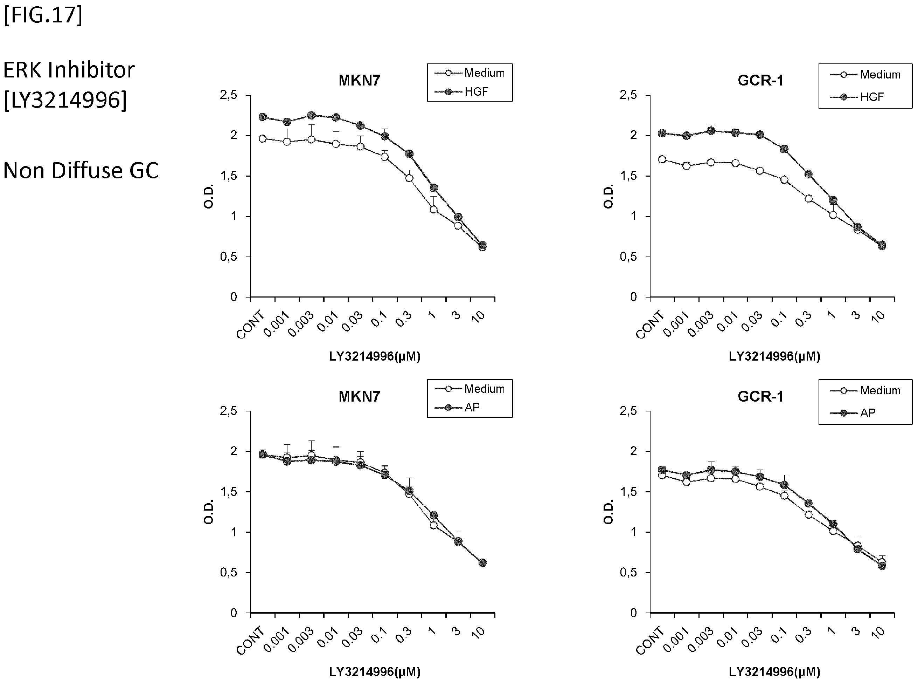

FIG. 17 shows proliferation inhibitory effects of LY3214996 (ERK inhibitor) on non-diffuse gastric cancer cells. "HGF" represents cell proliferation when HGF and LY3214996 were added; "AP" represents cell proliferation when amphiregulin and LY3214996 were added; and "Medium" represents cell proliferation when LY3214996 was added without HGF and AP (base line proliferation).

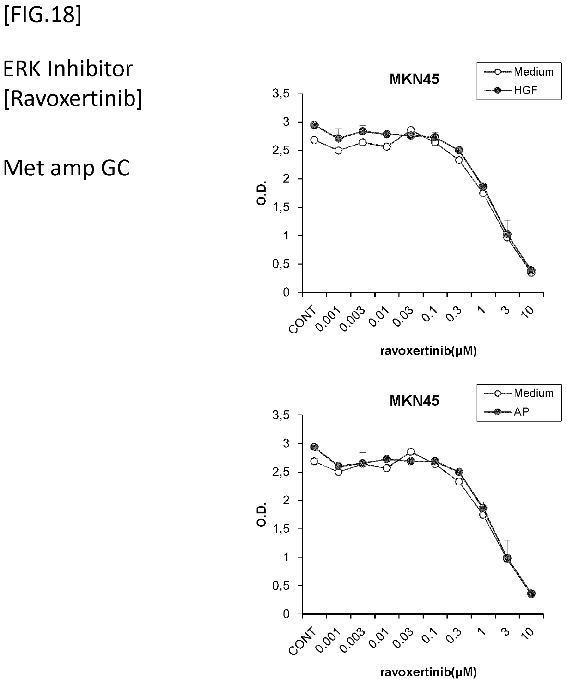

FIG. 18 shows antitumor effects of ravoxertinib (ERK inhibitor) on cMet gene-amplified gastric cancer cells. "HGF" represents cell proliferation when HGF and ravoxertinib were added; "AP" represents cell proliferation when amphiregulin and ravoxertinib were added; and "Medium" represents cell proliferation when ravoxertinib was added without HGF and AP (base line proliferation).

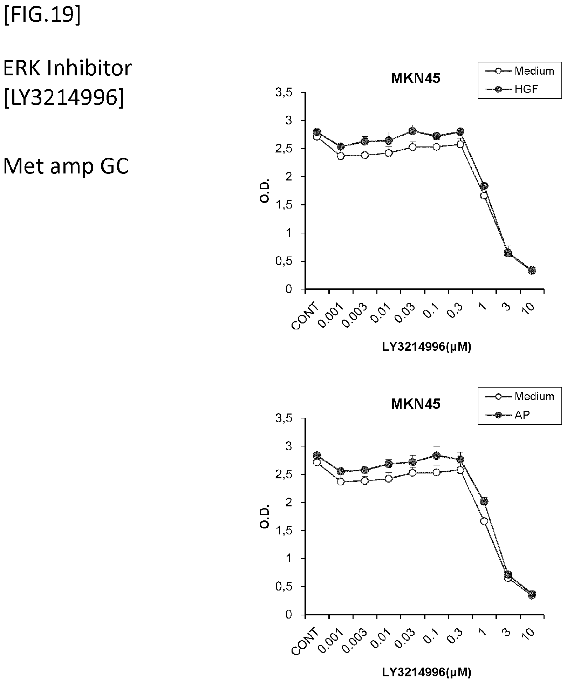

FIG. 19 shows proliferation inhibitory effects of LY3214996 (ERK inhibitor) on cMet gene-amplified gastric cancer cells. "HGF" represents cell proliferation when HGF and LY3214996 were added; "AP" represents cell proliferation when amphiregulin and LY3214996 were added; and "Medium" represents cell proliferation when LY3214996 was added without HGF and AP (base line proliferation).

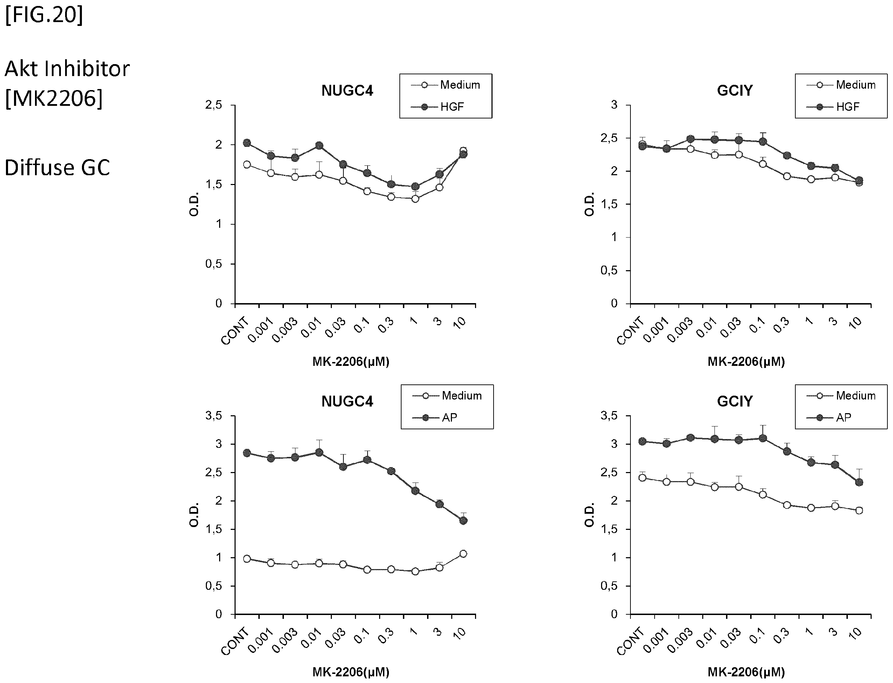

FIG. 20 shows antitumor effects of MK2206 (Akt inhibitor) on diffuse gastric cancer cells. "HGF" represents cell proliferation when HGF and MK2206 were added; "AP" represents cell proliferation when amphiregulin and MK2206 were added; and "Medium" represents cell proliferation when MK2206 was added without HGF and AP (base line proliferation).

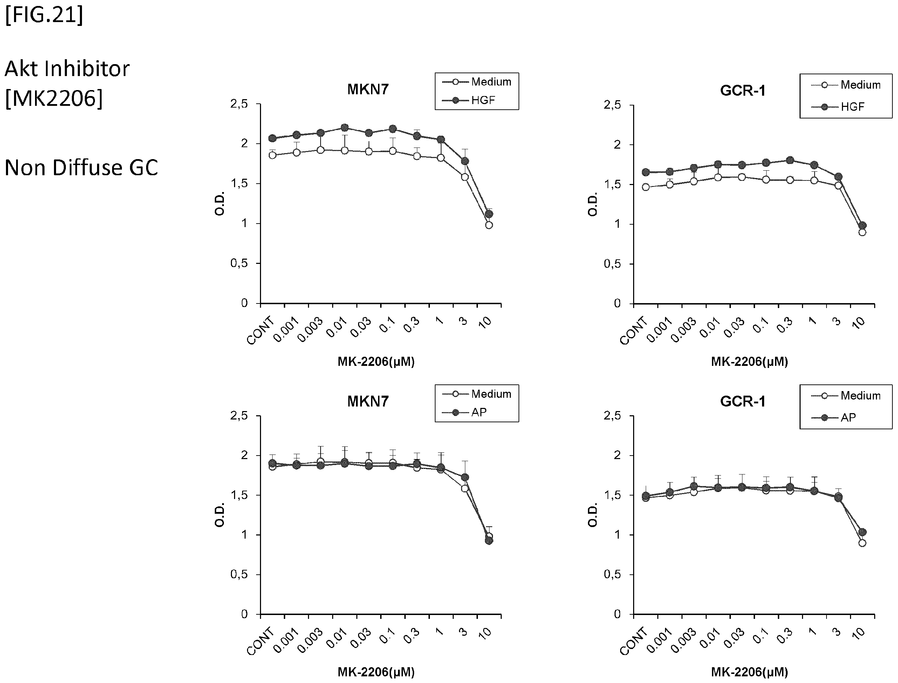

FIG. 21 shows antitumor effects of MK2206 (Akt inhibitor) on non-diffuse gastric cancer cells. "HGF" represents cell proliferation when HGF and MK2206 were added; "AP" represents cell proliferation when amphiregulin and MK2206 were added; and "Medium" represents cell proliferation when MK2206 was added without HGF and AP (base line proliferation).

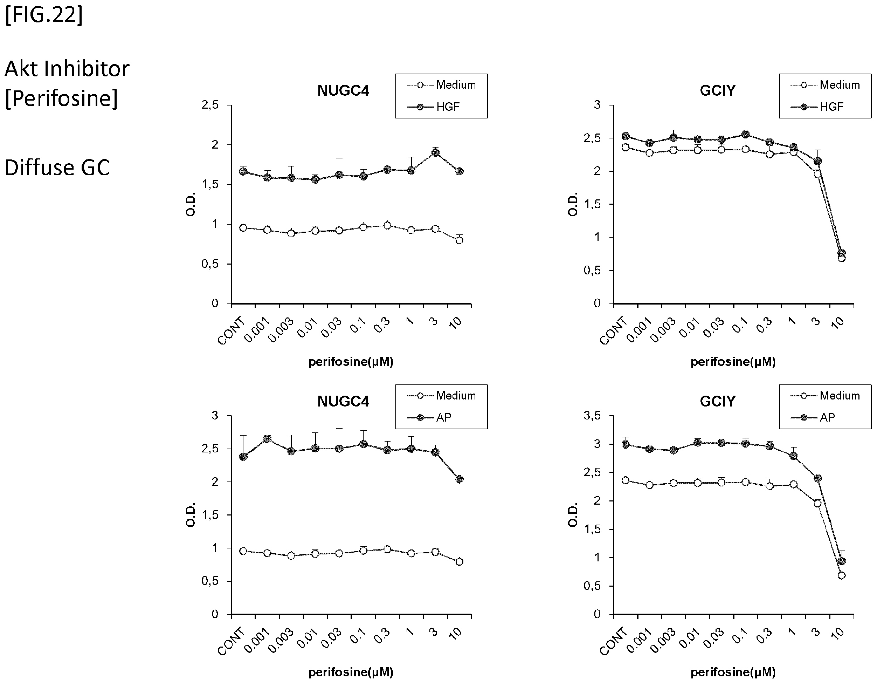

FIG. 22 shows antitumor effects of perifosine (Akt inhibitor) on diffuse gastric cancer cells. "HGF" represents cell proliferation when HGF and perifosine were added; "AP" represents cell proliferation when amphiregulin and perifosine were added; and "Medium" represents cell proliferation when perifosine was added without HGF and AP (base line proliferation).

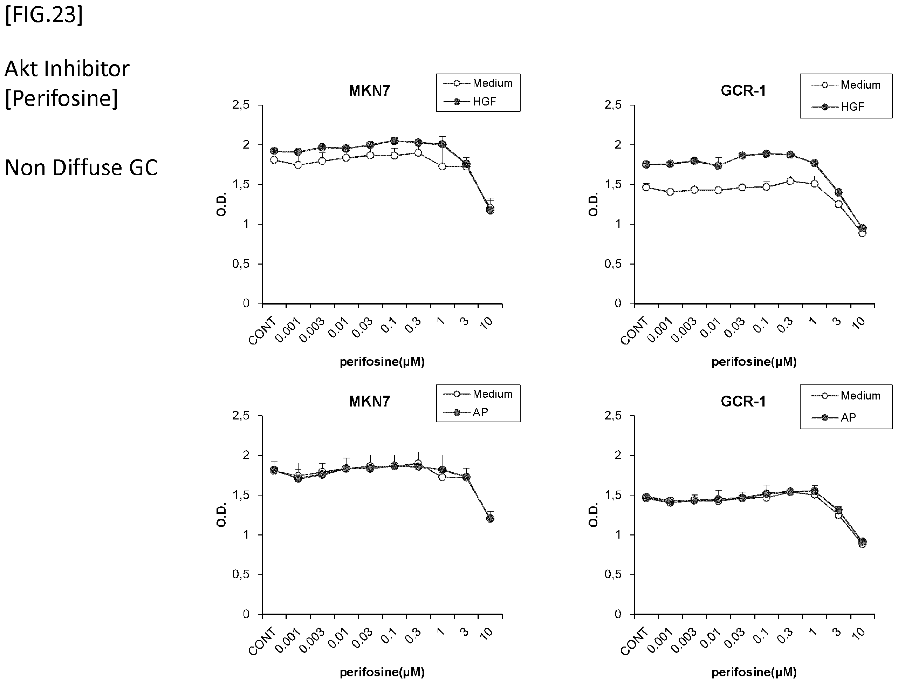

FIG. 23 shows antitumor effects of perifosine (Akt inhibitor) on non-diffuse gastric cancer cells. "HGF" represents cell proliferation when HGF and perifosine were added; "AP" represents cell proliferation when amphiregulin and perifosine were added; and "Medium" represents cell proliferation when perifosine was added without HGF and AP (base line proliferation).

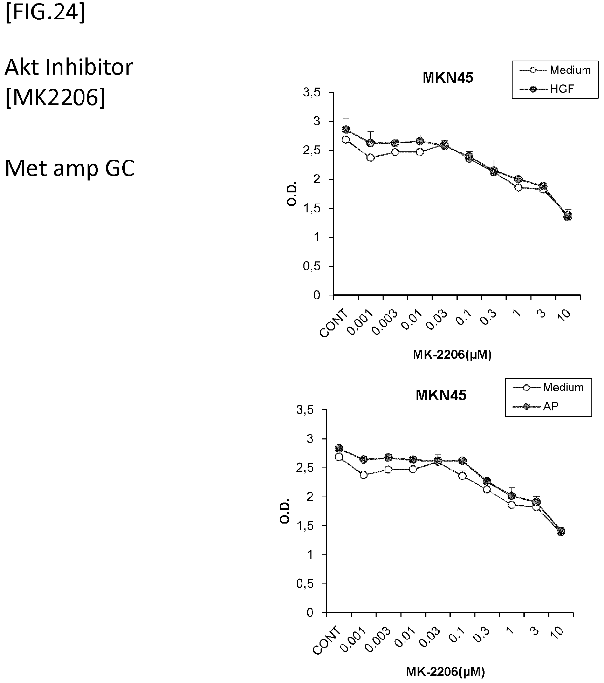

FIG. 24 shows antitumor effects of MK2206 (Akt inhibitor) on cMet gene-amplified gastric cancer cells. "HGF" represents cell proliferation when HGF and MK2206 were added; "AP" represents cell proliferation when amphiregulin and MK2206 were added; and "Medium" represents cell proliferation when MK2206 was added without HGF and AP (base line proliferation).

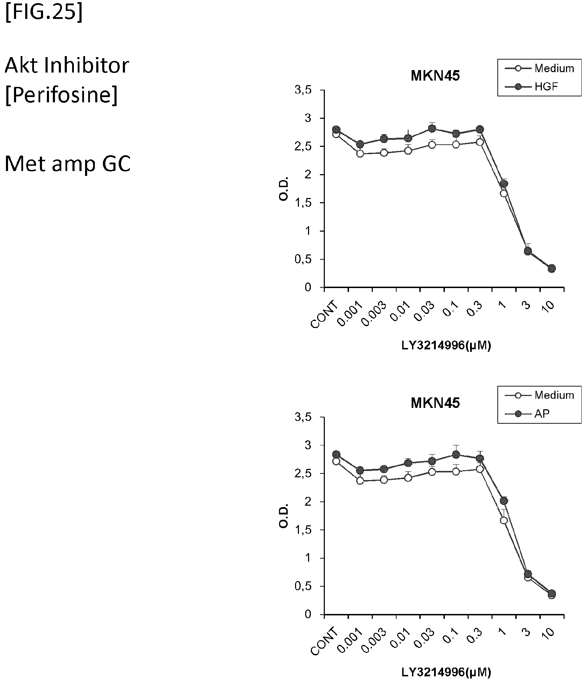

FIG. 25 shows antitumor effects of perifosine (Akt inhibitor) on cMet gene-amplified gastric cancer cells. "HGF" represents cell proliferation when HGF and perifosine were added; "AP" represents cell proliferation when amphiregulin and perifosine were added; and "Medium" represents cell proliferation when perifosine was added without HGF and AP (base line proliferation).

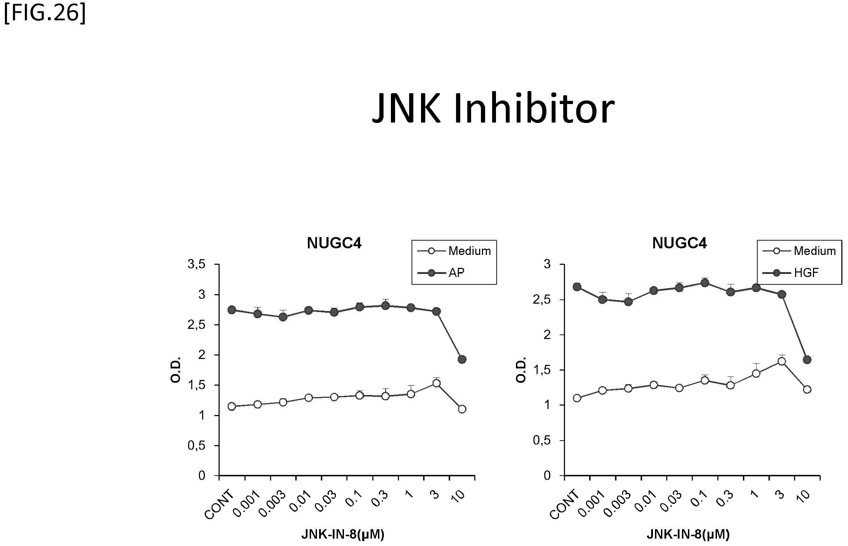

FIG. 26 shows antitumor effects of JNK-IN-8 (JNK inhibitor) on diffuse gastric cancer cells. "HGF" represents cell proliferation when HGF and JNK-IN-8 were added; "AP" represents cell proliferation when amphiregulin and JNK-IN-8 were added; and "Medium" represents cell proliferation when JNK-IN-8 was added without HGF and AP (base line proliferation).

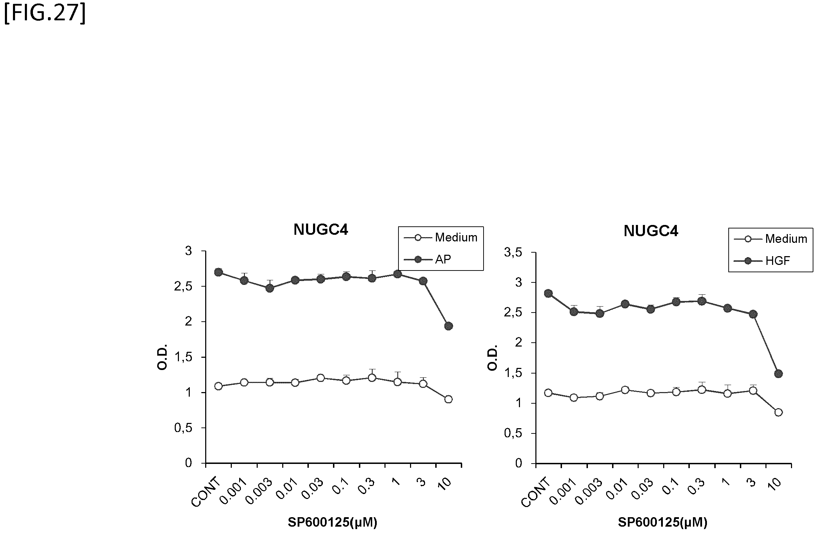

FIG. 27 shows antitumor effects of SP600125 (JNK inhibitor) on diffuse gastric cancer cells. "HGF" represents cell proliferation when HGF and SP600125 were added; "AP" represents cell proliferation when amphiregulin and SP600125 were added; and "Medium" represents cell proliferation when SP600125 was added without HGF and AP (base line proliferation).

### Mode for carrying out the Invention

The content of the present invention will be described in detail as follows.

In the description of the present specification, the therapeutic agent means a medical agent for treating a human or a mammal other than human suffering from a disease. Such a therapeutic agent contains, as a main component, (i) an MEK inhibitor or (ii) an MEK inhibitor and an mTOR inhibitor. By macroscopic morphology, the gastric cancer is classified into Types 1 to 5, in which Type 3 and Type 4 are considered as diffuse gastric cancer, and Type 4 is considered as scirrhous gastric cancer.

In the present invention, the MEK inhibitor may be any MEK inhibitor that exhibits MEK inhibitory effect to inhibit the MAPK/ERK signal transduction cascade that involves with cell proliferation. Examples thereof may include trametinib, refametinib (RDEA119, Bay 86-9766), cobimetinib (GDC-0973, RG7420), binimetinib (MEK162, ARRY-162, ARRY-438162), AZD6244 (selumetinib), AZD8330, pimasertib (AS-703026), PD0325901, PD184352 (CI-1040), pharmacologically acceptable salts thereof, and solvates thereof. Preferred examples may include trametinib, AZD6244 (selumetinib), pharmacologically acceptable salts thereof, and solvates thereof. More preferred examples may include trametinib, a pharmacologically acceptable salt thereof, and a solvate thereof. Among them, a trametinib dimethyl sulfoxide may be specifically preferred. The trametinib dimethyl sulfoxide is an active component of Mekinist (trademark) tablets.

In the present invention, the mTOR inhibitor may be any mTOR inhibitor that exhibits mTOR inhibitory effect. Examples thereof may include everolimus, AZD805, rapamycin and analogs thereof, RAD001, CCI-779, temsirolimus, AP23573, AZD8055, WYE-354, WYE-600, WYE-687, Pp121, dactolisib (BEZ235, NVP-BEZ235), ridaforolimus (deforolimus, MK-8669), sapanisertib (INK 128, MLN0128), omipalisib (GSK2126458, GSK458), vistusertib (AZD2014), torin 2, pharmacologically acceptable salts thereof, and solvates thereof. Preferred examples may include everolimus, AZD805, pharmacologically acceptable salts thereof, and solvates thereof. More preferred examples may include everolimus and a pharmacologically acceptable salt thereof. Among them, everolimus may be specifically preferred. The everolimus is an active component of Afinitor (trademark) tablets.

In the description of the present specification, the salt may be any salt that forms a salt with the MEK inhibitor or the mTOR inhibitor of the present invention and is pharmacologically acceptable. Examples may include inorganic acid salts, organic acid salts, inorganic base salts, organic base salts, acidic amino acid salts, and basic amino acid salts.

Examples of the inorganic acid salt may include a hydrochloride, a hydrobromide, a sulfate, a nitrate, and a phosphate.

Examples of the organic acid salt may include an acetate, a succinate, a fumarate, a maleate, a tartrate, a citrate, a lactate, a stearate, a benzoate, a methanesulfonate, an ethanesulfonate, a p-toluenesulfonate, and a benzenesulfonate.

Examples of the inorganic base salt may include alkali metal salts such as a sodium salt and a potassium salt, alkaline earth metal salts such as a calcium salt and a magnesium salt, an aluminum salt, and an ammonium salt.

Examples of the organic base salt may include a diethylamine salt, a diethanolamine salt, a meglumine salt, and an N,N'-dibenzylethylenediamine salt.

Examples of the acidic amino acid salt may include an aspartate and a glutamate.

Examples of the basic amino acid salt may include an arginine salt, a lysine salt, and an ornithine salt.

In the description of the present specification, examples of the solvate may include a hydrate and a non-aqueous solvate. Examples of the solvent may include water, alcohols (such as methanol, ethanol, and n-propanol), dimethyl sulfoxide, and dimethylformamide.

In the present invention, the MEK inhibitor or the mTOR inhibitor may be in a crystalline form or an amorphous form. When crystal polymorphisms are present, such an inhibitor may be in a single crystalline form or in a mixture of multiple crystalline forms.

The therapeutic agent for treating diffuse gastric cancer of the present invention may be a preparation for oral administration including solid preparations such as tablets, granules, fine granules, powders, and capsules, liquids, jellies, and syrups. The therapeutic agent for treating diffuse gastric cancer of the present invention may also be a preparation for parenteral administration including injections, suppositories, ointments, and cataplasms.

To produce a preparation for oral administration, as needed, a pharmaceutically acceptable carrier such as a diluent, a binder, a disintegrator, a lubricant, and a colorant may be added to the MEK inhibitor or the mTOR inhibitor of the present invention. For tablets, granules, powders or capsules, coating may be applied, as needed.

Examples of the diluent may include lactose, cornstarch, white soft sugar, glucose, sorbitol, crystalline cellulose, and silicon dioxide. Examples of the binder may include polyvinyl alcohol, ethyl cellulose, methyl cellulose, gum arabic, hydroxypropyl cellulose, and hydroxypropyl methyl cellulose. Examples of the lubricant may include magnesium stearate, talc, and silica. Examples of the colorant may include titanium oxide, iron sesquioxide, yellow ferric oxide, cochineal, carmine, and riboflavin. Examples of the flavoring agent may include cocoa powder, ascorbic acid, tartaric acid, peppermint oil, borneol, and powdered cinnamon bark. For these tablets or granules, coating may be applied, as needed.

To produce injections (such as those for intravenous administration, for intramuscular administration, for subcutaneous administration, for intraperitoneal administration), injections can be produced in usual procedure by adding, as needed, a pharmaceutically acceptable carrier such as a pH adjuster, a buffer, a suspending agent, a solubilizing agent, an antioxidant, a preservative (antiseptic agent), and a tonicity agent to the MEK inhibitor or the mTOR inhibitor of the present invention. The resulting product may be freeze-dried into a freeze-dried preparation to be dissolved at the time of use.

Examples of the suspending agent may include methyl cellulose, polysorbate 80, hydroxyethyl cellulose, gum arabic, tragacanth powder, sodium carboxymethyl cellulose, and polyoxyethylene sorbitan monolaurate.

Examples of the solubilizing agent may include polyoxyethylene hydrogenated castor oil, polysorbate 80, nicotinamide, polyoxyethylene sorbitan monolaurate, macrogol, and glycerol fatty acid ester.

Examples of the stabilizer may include sodium sulfite and sodium metasulfite. Examples of the preservative may include methyl p-hydroxybenzoate, ethyl p-hydroxybenzoate, sorbic acid, phenol, cresol, and chlorocresol.

The therapeutic agent for treating diffuse gastric cancer of the present invention can be produced using the MEK inhibitor and/or the mTOR inhibitor of the present invention in accordance with the known method such as a method according to General Rules for Preparations in the Japanese Pharmacopoeia, Seventeenth Edition.

In the therapeutic agent for treating tumor of the present invention, the MEK inhibitor and the mTOR inhibitor of the present invention may be separately formulated, which may be administered simultaneously or separately. The two preparations may be placed in a single package to give what is called a kit preparation. The two inhibitors may be contained in a single preparation.

In the therapeutic agent for treating diffuse gastric cancer of the present invention, each dose of the MEK inhibitor and the mTOR inhibitor can be appropriately selected depending on the severity of symptoms, a patient's age, a gender, a body weight, a differential sensitivity, an administration method, an administration timing, an administration interval, the type of a medical preparation, and the like.

The MEK inhibitor of the present invention can be administered in accordance with the known clinical performance. When the MEK inhibitor of the present invention is orally administered, the daily dose of the MEK inhibitor for an adult (body weight: 60 kg) may be 100 micro g to 10 g, preferably 500 micro g to 10 g, more preferably 1 mg to 1 g, and even more preferably 1 mg to 10 mg.

When the MEK inhibitor of the present invention is a trametinib dimethyl sulfoxide, the daily dose thereof is 1 to 2 mg in one embodiment.

The daily dose of the MEK inhibitor of the present invention can be divided and administered once to three times a day.

The mTOR inhibitor of the present invention can be administered in accordance with the known clinical performance. When the mTOR inhibitor of the present invention is orally administered, the daily dose of the mTOR inhibitor for an adult (body weight: 60 kg) may be 100 micro g to 10 g, preferably 500 micro g to 10 g, more preferably 1 mg to 1 g, and even more preferably 5 mg to 20 mg.

When the mTOR inhibitor of the present invention is everolimus, the daily dose thereof is 10 mg in one embodiment.

The daily dose of the mTOR inhibitor of the present invention can be divided and administered once to three times a day.

The present invention will be described in more detail with reference to examples as follows, but the examples are not intended to limit the scope of the invention.

### Examples

### Example 1

The effect of trametinib on proliferation of diffuse gastric cancer cell was examined.

Each diffuse gastric cancer cell strain of NUGC4 (obtained from Health Science Research Resources Bank (Osaka, Japan)) and GCIY (obtained from Riken BioResource Research Center (Tsukuba, Japan)) was cultured and passaged in an RPMI1640 culture medium containing 10% fetal calf serum, 100 units/mL penicillin, and 100 units/mL streptomycin antibiotic, and was used for the following experiments. Mycoplasma infection of cultured cells was regularly checked by MycoAlert Mycoplasma Detection kit (Lonza).

Cell proliferation was measured by Cell Counting Kit-8 (CCK-8) (Dojindo Laboratories, Kumamoto, Japan) (M. Ishiyama, et al., Talenta 44; 1299: 1997). Cells were seeded at 100 micro L/well at a concentration of 5 × 10⁴ cells/mL and were cultured in an incubator at 37°C for 24 hours. To each well, HGF (R&D systems) (50 ng/mL) or amphiregulin (R&D Systems (Minneapolis, MN)) (100 ng/mL) and trametinib (Selleck Chemicals (Houston, TX)) (0.001 to 10 micro M) were then added, and the cells were further cultured in an incubator at 37°C for 72 hours. To each well, 10 micro L of a WST-8 solution was added, which was incubated at 37°C for 4 hours. Absorbance was measured at a wavelength of 450 nm by a microplate reader (BIO-RAD Laboratories, Inc.). A similar experiment was repeated three times in triplets.

As a result, trametinib suppressed the HGF induced cell proliferation, the amphiregulin induced cell proliferation, and the base line cell proliferation in each of NUGC4 and GCIY. In particular, in NUGC4, trametinib strongly suppressed the HGF induced cell proliferation, the amphiregulin induced cell proliferation, and the base line cell proliferation, in a dose-dependent manner from a very low concentration below 0.001 micro m. In GCIY, proliferation inhibitory effect on the amphiregulin induced proliferation was observed from a concentration of 0.03 micro M or more. The results are shown in FIG. 2 and Table 1.

**[Table 1]**

| Trametinib IC50 (micro M) | | | |
|---|---|---|---|
| | HGF | amphiregulin | Base line |
| NUGC4 | 0.1 | 0.3 | 0.001 ≥ |
| GCIY | 0.1 | 10 | 0.1 |

### Example 2

Effects of trametinib on proliferation of non-diffuse gastric cancer cell were examined by the similar method to that in Example 1.

Human non-diffuse, well-differentiated gastric cancer cells used were MKN7 (Health Science Research Resources Bank (Osaka, Japan)) and GCR1 (Cancer Research Institute, Kanazawa University).

As a result, in each of MKN7 and GCR1, trametinib suppressed the HGF induced cell proliferation, the AP induced cell proliferation, and the base line (without HGF or amphiregulin) cell proliferation, in a dose-dependent manner from a very low concentration below 0.001 micro M, but the effects were weaker than on the diffuse gastric cancer cells. The results are shown in FIG. 3 and Table 2.

**[Table 2]**

| Trametinib IC50 (micro M) | | | |
|---|---|---|---|
| | HGF | amphiregulin | Base line |
| MKN7 | 0.1 | 0.3 | 0.1 |
| GCR1 | 0.1 | 0.3 | 0.1 |

### Example 3

Effects of trametinib on proliferation of cMet gene-amplified gastric cancer cell were examined by the similar method to that in Example 1.

As the cMet gene-amplified gastric cancer cells, cMet gene-amplified gastric cancer cell strain MKN45 (Health Science Research Resources Bank (Osaka, Japan)) was used.

As a result, trametinib hardly exhibited HGF induced cell proliferation and AP induced cell proliferation in MKN45, and trametinib exhibited marked cell proliferation inhibition from a very low concentration below 0.001 micro M. The results are shown in FIG. 4 and Table 3.

**[Table 3]**

| Trametinib IC50 (micro M) | | | |
|---|---|---|---|
| | HGF | amphiregulin | Base line |
| MKN45 | 0.001 | 0.001 | 0.001 |

### Example 4

Effects of everolimus (Selleck Chemicals) on diffuse gastric cancer cells, cMet gene-amplified gastric cancer cells, and non-diffuse gastric cancer cells were examined by the similar method to that in Example 1.

As a result, in each examined cell strain of the diffuse gastric cancer cells (NUGC4 and GCIY), the cMet gene-amplified gastric cancer cells (MKN45), and the non-diffuse gastric cancer cells (MKN7 and GCR1), everolimus exhibited certain inhibitory effects on HGF induced cell proliferation, amphiregulin induced cell proliferation, and base line cell proliferation at a very low concentration below 0.001 micro M, but did not exhibit obvious inhibitory effects at a concentration of 0.001 micro M or more except for a high concentration of 10 micro M. The results are shown in FIG. 5 and FIG. 6.

### Comparative Example 1

Effects of an Akt inhibitor, AZD5363 (Selleck Chemicals), on proliferation of diffuse gastric cancer cells (NUGC4) were examined by the similar method to that in Example 1.

As a result, on the HGF induced cell proliferation, no inhibitory effect was exhibited except for a high concentration of 10 micro M, and on the amphiregulin induced cell proliferation, a slight inhibitory effect was exhibited at a high concentration of 1 micro M or more. No inhibitory effect was exhibited on the base line cell proliferation. As above, the inhibitory effect of the Akt inhibitor (AZD5363) was even lower than the inhibitory effect of everolimus. The results are shown in FIG. 7.

### Example 5

The presence or absence of proliferation inhibitory effects of combined use of trametinib and everolimus was examined by the similar method to that in Example 1. Everolimus was so added as to give a concentration of 1 micro M.

In diffuse gastric cancer cells (NUGC4 and GCIY), the cell proliferation inhibitory effect of trametinib was synergistically improved by addition of everolimus. In contrast, in non-diffuse gastric cancer cells (MKN7 and GCR1), no combined effect was exhibited. The results are shown in FIGS. 8 to 10 and Table 4.

**[Table 4]**

| Trametinib IC50 (micro M) | | | |
|---|---|---|---|
| | HGF | amphiregulin | Base line |
| NUGC4 | 0.01 | 0.03~0.1 | 0.001> |
| GCIY | 0.1 | 0.3 | 0.03 |
| MKN7 | 0.1 | 0.1 | 0.03 |
| GCR1 | 0.1 | 0.1 | 0.03 |

### Example 6

To a Balb/c nu/nu mouse (6 weeks old), diffuse gastric cancer cells (NUGC4 cells, 2 x 10⁶ cells in 200 micro L PBS) were transplanted by intraperitoneal administration. On 21 days after transplantation, ascites was observed, and oral administration was started with trametinib (0.1 mg/kg body weight or 0.3 mg/kg body weight), everolimus (1.5 mg/kg body weight), combined use of trametinib (0.1 mg/kg body weight) and everolimus (1 mg/kg body weight), combined use of trametinib (0.1 mg/kg body weight) and everolimus (1 mg/kg body weight), combined use of trametinib (0.3 mg/kg) and everolimus (1 mg/kg), or combined use of trametinib (0.3 mg/kg body weight) and everolimus (5 mg/kg body weight). Each of the trametinib and the everolimus was dissolved in a solution (7% DMSO, 13% Tween80, 80% Otsuka glucose injection (5%), HCl equimolar to each compound). To a control group, only a solution containing no medicinal agent was administered. Administration was continued for 4 weeks, followed by observation for the following 4 weeks without administration of the medicinal agent. In the control mouse group without treatment, all the mice died with marked ascites formation within 7 weeks after the transplantation of tumor cells. In the trametinib administered group, the everolimus administered group, and the combined administration group, prolonged survival times were observed as compared with the control group. The results are shown in FIG. 11.

### Example 7

In order to examine combined effects of an MEK inhibitor and an mTOR inhibitor other than the combined effects of trametinib (MEK inhibitor) and everolimus (mTOR inhibitor), the presence or absence of proliferation inhibitory effects of combined use of an MEK inhibitor, PD0325901 (Selleck Chemicals), and an mTOR inhibitor, temsirolimus (Selleck Chemicals), was examined by the similar method to that in Example 1. Temsirolimus was so added as to give a concentration of 1 micro M. As a result, in diffuse gastric cancer cells (NUGC4), the cell proliferation inhibitory effect of PD0325901 was synergistically improved by addition of temsirolimus. In contrast, in non-diffuse gastric cancer cells (MKN7), no combined effect was exhibited. The results are shown in FIG. 12.

### Example 8

For the same purpose as in Example 7, the presence or absence of proliferation inhibitory effects of combined use of an MEK inhibitor, pimasertib (Selleck Chemicals), and an mTOR inhibitor, rapamycin (Selleck Chemicals), was examined by the similar method to that in Example 1. Rapamycin was so added as to give a concentration of 1 micro M. As a result, in diffuse gastric cancer cells (NUGC4), the cell proliferation inhibitory effect of trametinib was synergistically improved by addition of everolimus. In contrast, in non-diffuse gastric cancer cells (MKN7), no combined effect was exhibited. The results are shown in FIG. 13.

### Comparative Example 2

Effects of an ERK inhibitor, ravoxertinib (Selleck Chemicals) or LY3214996 (Selleck Chemicals), on the proliferation of diffuse gastric cancer cells (NUGC4 or GCIY), non-diffuse gastric cancer cells (MKN7 or GCR1), or cMet gene-amplified gastric cancer cells (MKN45) were examined by the similar method to those in Example 1, Example 2, and Example 3.

As a result, the ERK inhibitors did not exhibit obvious inhibitory effects in the diffuse gastric cancer cells, the non-diffuse gastric cancer cells, and the cMet gene-amplified gastric cancer cells on the HGF induced cell proliferation, the amphiregulin induced cell proliferation, and the base line cell proliferation. The results are shown in FIG. 14 to FIG. 19.

### Comparative Example 3

Effects of an Akt inhibitor, MK2206 (Selleck Chemicals) or perifosine (Selleck Chemicals), on the proliferation of diffuse gastric cancer cells (NUGC4 or GCIY), non-diffuse gastric cancer cells (MKN7 or GCR1), or cMet gene-amplified gastric cancer cells (MKN45) were examined by the similar method to that in Comparative Example 1.

As a result, the Akt inhibitors did not exhibit obvious inhibitory effects in the diffuse gastric cancer cells, the non-diffuse gastric cancer cells, and the cMet gene-amplified gastric cancer cells on the HGF induced cell proliferation, the amphiregulin induced cell proliferation, and the base line cell proliferation. The results are shown in FIG. 20 to FIG. 25.

### Comparative Example 4

Effects of JNK inhibitors which is one of the MAPK signal pathways, JNK-IN-8 (Selleck Chemicals) and SP600125 (Selleck Chemicals), on the proliferation of diffuse gastric cancer cells (NUGC4) were examined by the similar method to that in Example 1.

As a result, the JNK inhibitors did not exhibit obvious inhibitory effects in the diffuse gastric cancer cells on the HGF induced cell proliferation, the amphiregulin induced cell proliferation, and the base line cell proliferation. The results are shown in FIGS. 26 and 27.

### Industrial Applicability

The therapeutic agent for treating diffuse gastric cancer and the therapeutic agent for treating cMet gene-amplified gastric cancer each containing (i) the MEK inhibitor or (ii) the MEK inhibitor and the mTOR inhibitor of the present invention can be used as a therapeutic agent for (i) diffuse gastric cancer typified by scirrhous gastric cancer or as a therapeutic agent for (ii) cMet gene-amplified gastric cancer.

## Claims

1. A therapeutic agent for treating diffuse gastric cancer, which comprises an MEK inhibitor.

2. The therapeutic agent according to claim 1, wherein the MEK inhibitor is trametinib, a pharmacologically acceptable salt thereof, or a solvate thereof.

3. The therapeutic agent according to claim 1 or 2, wherein the MEK inhibitor is a trametinib dimethyl sulfoxide.

4. A therapeutic agent for treating diffuse gastric cancer, which comprises an MEK inhibitor and an mTOR inhibitor.

5. The therapeutic agent according to claim 4, wherein the MEK inhibitor is trametinib, a pharmacologically acceptable salt thereof, or a solvate thereof.

6. The therapeutic agent according to claim 4 or 5, wherein the MEK inhibitor is a trametinib dimethyl sulfoxide.

7. The therapeutic agent according to any one of claims 4 to 6, wherein the mTOR inhibitor is everolimus, a pharmacologically acceptable salt thereof, or a solvate thereof.

8. The therapeutic agent according to any one of claims 4 to 7, wherein the mTOR inhibitor is everolimus.

9. A therapeutic agent for treating diffuse gastric cancer, which comprises an MEK inhibitor, in which the therapeutic agent is to be administered in combination with an mTOR inhibitor.

10. The therapeutic agent according to claim 9, wherein the MEK inhibitor is trametinib, a pharmacologically acceptable salt thereof, or a solvate thereof.

11. The therapeutic agent according to claim 9 or 10, wherein the MEK inhibitor is a trametinib dimethyl sulfoxide.

12. The therapeutic agent according to any one of claims 9 to 11, wherein the mTOR inhibitor is everolimus, a pharmacologically acceptable salt thereof, or a solvate thereof.

13. The therapeutic agent according to any one of claims 9 to 12, wherein the mTOR inhibitor is everolimus.

14. The therapeutic agent according to any one of claims 1 to 13, wherein the diffuse gastric cancer is scirrhous gastric cancer.

15. A therapeutic agent for treating cMet gene-amplified gastric cancer, which comprises an MEK inhibitor.

16. The therapeutic agent according to claim 15, wherein the MEK inhibitor is trametinib, a pharmacologically acceptable salt thereof, or a solvate thereof.

17. The therapeutic agent according to claim 15 or 16, wherein the MEK inhibitor is a trametinib dimethyl sulfoxide.
